# EUROPEAN PATENT SPECIFICATION

(11) **EP 2 749 228 B1**
(45) Date of publication and mention of the grant of the patent: **07.03.2018**
(21) Application number: 14162188.8
(22) Date of filing: 01.08.2008
(51) Int. Cl.: A61B 8/00, G01S 7/52, G01S 7/521, H05K 5/00, G06F 1/16

(54) **Portable ultrasound system**
Tragbares Ultraschallsystem
Système d'ultrasons portable

(43) Date of publication of application: 02.07.2014
(62) Divisional of application: 08786802.2
(73) Proprietor: Esaote S.p.A., 16152 Genova (IT)
(72) Inventor: Willems, John, 6227 Aj Maastricht (NL); Geijsen, Joop, 6227 Aj Maastricht (NL)
(74) Representative: Karaghiosoff, Giorgio Alessandro

(56) References cited:
- EP-A1- 1 925 257
- WO-A1-2006/040697
- US-A- 5 609 485
- US-A1- 2007 276 250
- US-A1- 2008 119 731
- EIDHEIM ET AL: "Real-time analysis of ultrasound images using GPU", INTERNATIONAL CONGRESS SERIES, EXCERPTA MEDICA, AMSTERDAM, vol. 1281, 1 May 2005 (2005-05-01), pages 284-289, XP005081682, ISSN: 0531-5131

## Description

The invention relates to portable ultrasonic system according to the preamble of claim 1. Ultrasonic systems or devices of the above mentioned kind are known, e.g. from US 2008/0119731 A1, and are the result of advances in the underlying technology which have permitted miniaturization of necessary electronics. The ultrasound industry continues to reduce the size and weight of available diagnostic equipment. The smaller and lighter equipment has improved portability however existing portable ultrasound technology is not without its limitations.

One shortcoming of some portable systems is that the need to hold onto or otherwise manipulate the equipment with both hands during use hinders the ability of a person to productively use the system in some situations. For example, document U.S. Pat. No. 5,590,658 discloses an ultrasound system including a lap top computer with the capability of displaying ultrasound images obtained with a handheld scan head. With this kind of system, in order to ensure the display is visible at all times during acquisition of the ultrasound signals with the scan head, the user must either hold the lap top computer, or change the position of the lap top to face the user who may be moving around to position the scan head in a suitable location during the imaging session. As a result, the system user's hand not being used to manipulate the scan head is often nonetheless occupied and not always free to assist with the medical procedure.

The portable ultrasound systems disclosed in U.S. Pat. Nos. 5,722,412 and 5,738,099, are of the kind so called "hand held" and integrate the display and the transducer in the same unit. While in essence permitting the scan head and the display to be held in one hand, these systems suffer from a different shortcoming. In particular, in order for the ultrasound user to get the desired view, it is frequently necessary to move the transducer repeatedly to different regions of the patient and to different orientations. In so doing, the displays of these systems may not be sufficiently visible to a user unless that user moves about, possibly into awkward positions.

A further kind of portable ultrasound systems is disclosed in document US 6 126 608. This kind of devices comprise a scan head and a main unit. Circuitry for forming the image based on the ultrasound signals collected by the scan head. A display screen which is carried by the main unit and on which the ultrasound image is displayed. The main unit is relatively small and is provided in combination with an attachment member which is mountable around a forearm of the system user such that said display is visible to the system user during use. The said attachment member extending toward a hand of the system user at the end of the forearm and comprising a digit-accommodating opening.

Although this ultrasound system overcomes the shortcomings of the above mentioned known systems, by leaving the hand free to carry out help functions during imaging, permitting at the same time to have the display always optimally directed against the user, so that the said user can have the best view of the display, there are still shortcomings which consist principally in the fact that:

The display is very small in size.

The hand at the arm to which the main unit is attached cannot carry out any task or function connected to the device such as controlling the device by means of control buttons or the like. These tasks must be carried out by the hand carrying the probe so that in certain situations the scanning process must be interrupted or even aborted due to the fact that some controls has to be activated on the main unit.

As a general drawback of the increasing miniaturisation trend of the ultrasound systems consists in the fact that the said portable systems are limited to certain specific applications of the ultrasound imaging technique and that in order to have systems being able to carry put different kinds of applications of the ultrasound imaging technique bigger and less portable devices are still needed.

Thus, it would be desirable to provide a portable ultrasound system which overcomes these and other shortcomings of the prior art indicated above.

The present invention provides a portable ultrasound system of the kind disclosed above according to claim 1. The main unit case has the form of a flat parallelepiped and in which the front side is formed substantially entirely by a so called touch screen.

In particular it has two larger sides which form the front and the back side and which are connected by smaller sides being the lateral sides.

So preferably no key or button or other kind of control are provided on the sides of the case of the main unit.

In a particular embodiment the front and the back sides of the case of the main unit are rectangular.

According to an embodiment (not claimed), the main unit has a case in the form of a tablet computer, having a front side formed by a display and a rear side and having a handle, the said handle being formed by an elongated member which is secured to the case of the main unit by cantilevered arms protruding from the case in a direction of the rear side of the case of the main unit and terminating behind the said rear side so that the elongated member forming the handle extends at a certain distance behind the rear side of the case of the main unit and is nearer to one of two opposite peripheral edges of the said case of the main unit in such a way that when one hand grasps the handle the case of the main unit leans against the forearm, the weight of the main unit being carried by the forearm and the hand having only the function of blocking the case of the main unit in position. The main unit comprises all of the hardware and peripherals of the ultrasound system needed for driving the ultrasound probe and for generating, processing and storing the images. Further hardware is preferably provided which is of general purpose such as communications units with other devices, networks or other external peripherals. The transducers which are housed in a probe together with some electronic components as it is used in the current traditional ultrasound probes.

According to still a further improvement (not claimed), the case of the main unit can be carried easily either in a so called portrait or in a so called landscape orientation of the display, this means according to two orientations of the display which are rotated of 90° one with respect to the other around an axis of rotation which is perpendicular to the display and passes through the centre of the display area.

When the display is rectangular in shape this means that the display is oriented with the longer edges along an horizontal direction (landscape orientation) in a first orientation and with the longer edges in a vertical direction (portrait orientation) in the second position.

In the said first orientation the elongated member forming the handle of the case of the main unit is also horizontal, while in the said second orientation the elongated member is oriented in such a way as being contained in a vertical plane.

In order to have a secure support of the main unit case in both the said first and second orientations, the length of the case of the main unit in the direction parallel to the longitudinal axis of the elongated member is less than twice the said average distance from the palm of the hand to the hollow of the elbow.

This features ensures that when the main unit is held in a position with the handle, i.e. the elongated member, in a vertical plane, the case is balanced and there is not a mayor contribution of the weight in the part of the case protruding over the zone at which the hand grasps the elongated member.

In one alternative, in order to further facilitate the carrying of the main unit, reducing the stress or work of the hand to firmly block the case grasping the handle and so allowing to use the said hand for pressing the keys of buttons arranged on the elongated member, the invention provides an ultrasound system with one or more of the features disclosed above having also an attachment member for said main unit case, said attachment member being mountable around a forearm of the system user such that said display is visible to the system user during use.

In its simplest form the attachment member is formed by at least one strap secured to the back side of the case of the main unit, i.e. the side oriented towards the forearm of the user, and which strap has two ends which carry means for securing the said two ends one to the other and for tightening the strap around the user forearm.

The straps are provided in an intermediate position between the elongated member and the side of the case of the main unit opposite to the one which is nearer to the elongated member.

Furthermore the at least one strap can be secured to the back of the case of the main unit in such a way as to be rotatable around an axis perpendicular to the said back side of the case of the main unit in such a way that the main unit can be rotated in one of the said first and second orientations of the display when the strap is tightly secured to the user arm.

According to a further development (not claimed), the attachment member is formed by a combination of a strap and an anatomic saddle like contact part against the fore arm. In this case, the said saddle like contact part is secured to the back of the case of the main unit in a rotatable way around an axis perpendicular to the back side of the said case.

Furthermore means may be provided for releasable stopping the said saddle like contact part in one angular position of the at least two angular positions corresponding to the above mentioned first and second orientations of the display.

This can be obtained by means of elastically releasable stopping means which are mounted in housings provided in the back side of the case of the said main unit or in the surface of the said saddle like contact part adhering to the said back side of the case, while the said means have one or more teeth or projection that are forced in a position protruding out of the surface of the said back side and can be pushed back in a position not protruding for the said back side, the said teeth or projections cooperating with notches provided in the surface of the saddle like contact part adhering the said back side and the said teeth or projections being placed on the same a circular path coaxial with the axis of rotation as the notches.

The display screen being of the touch screen kind the display screen is able of receiving commands by touching the screen and the different control keys, buttons, switches and cursors are in the form of graphic symbols representing the said keys, buttons, switches and cursors.

Selecting means are provided for selecting at least one specific application within a list of application which are available on the portable echographic system, means being provided for loading and executing a specific graphic user interface program which generates on the screen the images of only the keys, buttons, switches and cursors that are needed for carrying out the tasks related to the selected specific application.

Furthermore, according to a further feature, in the portable echographic system different electronic units for carrying out specific tasks are substituted by a central processing unit able of executing different programs and of carrying out different tasks, at least a memory being provided in which there are saved different programs for controlling the central processing unit in order to carry out different specific tasks related to the functions of an echographic system, one or more of the said different programs for carrying out specific tasks being loaded by the central processing unit and executed by it when a certain specific application has been selected by the user.

Advantageously a main control program can be loaded and executed by the central processing unit which program prints on the display screen the list of applications available with the said portable system and a further graphics user interface comprising the icons of keys, buttons, switches, cursors or similar control devices related to general functions, such as a menu for addressing the specific diagnostic or surgical applications and or menus for addressing configuration and adjustments options for the device which are of general purpose, and/or menus for carrying out maintenance or managing tasks of the apparatus and of the devices integrated in it such as file manager routines, memory drives control and maintenance routines, electronic units configuration and diagnostics, driver upgrade, system upgrade and further typical tasks needed for managing and for maintaining the efficiency of a computer system.

In particular as soon as the apparatus is started all the touch screen drivers and the menus graphic interface are loaded and executed.
The said main control program addresses then depending on the choice made with the help of the menus the programs for carrying out the specific tasks related to the applications or configuration and adjustment options chosen by the user.

According to a further advantageous feature of the present portable echographic system, a further program is saved in the memory and is available for being executed by the central processing unit, which program consist in a application specific tutorial for the user which is already provided in the echographic system in a ready to start condition.

In this case the main control program prints on the screen a graphic user interface with a key icon which addresses the said tutorial program and which key when touched by the user determines that the main control program loads the tutorial program and to execute the said program by the central processing unit.

Here also a main tutorial control program can be executed which loads a graphic user interface showing a list of options each one of which can be highlighted and chosen by means of the touch screen interface and which causes the corresponding specific program to be executed and the corresponding graphic user interface to be loaded and printed on the touch-screen display.
In order to spare electric power and maintain the weight limited without affecting the computational power of the system needed by the hardware for executing the programs and carrying out the tasks of the system, according to a further feature, in the present ultrasound diagnostic system a part of the tasks to be carried out by the central processing unit (CPU) are carried out by the graphic processing unit (GPU). In the ultrasound devices the GPU is not subjected to high operational stresses because the image processing itself is not a heavy operation. Computational power is normally needed to extract the image data from the signals received. Thus the GPU can be used as an aid to the CPU also for carrying out non typical tasks for the GPU. In the present invention the portable ultrasound system has a scan converter which is formed by a software to be executed by a processing unit while the graphic processing unit is used as the processing unit for executing the scan converter software.

The above features and the related advantages will become clear form the following description of preferred embodiments which are illustrated in the annexed drawings, in which:
Figure 1 is a perspective view of the main unit of an ultrasound system in its condition laying on a table and in its simplest configuration as a tablet PC.
Figure 2 illustrates the main unit of figure 1 in the condition being transported as a brief case.
Figure 3 illustrates a first condition of holding the main unit during use and corresponding to a panoramic orientation of the screen.
Figure 4 illustrates a second condition of holding the main unit during use and corresponding to a portrait orientation of the screen.
Figure 5 to 8 illustrates several views of the combination of the main unit and of a main unit table support which allows to position the main unit such that the screen is positioned approximately in a vertical position, while in fig 8 the main unit is separated from the support.
Fig. 9 illustrates the backside of the main unit to which a saddle like contact part is attached for securing the main unit to the arm and leaving the hand free from grasping the handle.
Fig. 10 illustrates a variant embodiment in which the elongated member forming the handle is doubled by attaching a second elongated member to the first one.
Fig. 11 illustrates a variant embodiment in which the main unit is attached with its back side to a docking unit.
Fig. 12 and 13 illustrates a variant embodiment in which the elongated member together with the two supporting arms are releasably secured to the case of the main unit.
Figure 14 and 15 illustrates the same views as figures 12 and 13 bit with an elongated member having a thicker cross section than the one of figures 12 and 13.
Fig. 16 illustrates a particular of an example of releasable connection means which can be used for attaching the different add-ons disclosed in the previous figures.
Fig. 17 illustrates an example of releasable electric connectors which can be used in combination with the said releasable connection means.
Fig. 18 is a block diagram of the software and hardware structure of the portable ultrasound system.
Fig. 19 and Fig. 20 illustrates an example of a particular embodiment of the connector which secures the elongated member to the case of the main unit and also an example of the therein integrated conductors for feeding the power signal to the main unit and or for feeding other electric signals generated for example by keys or buttons or similar devices provided on the elongated member.
Fig. 21 is a schematic example of the lay out of the elongated member which elongated member is void inside and has three chambers one of which is for the power feeding circuit for connecting the system to an external power source such as the electric network and also for a recharging unit for the battery packs while the other two chambers are for housing each one a battery pack.
Figures 22 to 25 are examples of different GUI (graphic user Interface) for different applications to be carried out by the ultrasound system according to the present invention and which graphic interfaces are generated by a software/hardware structure as disclosed in figure 18.

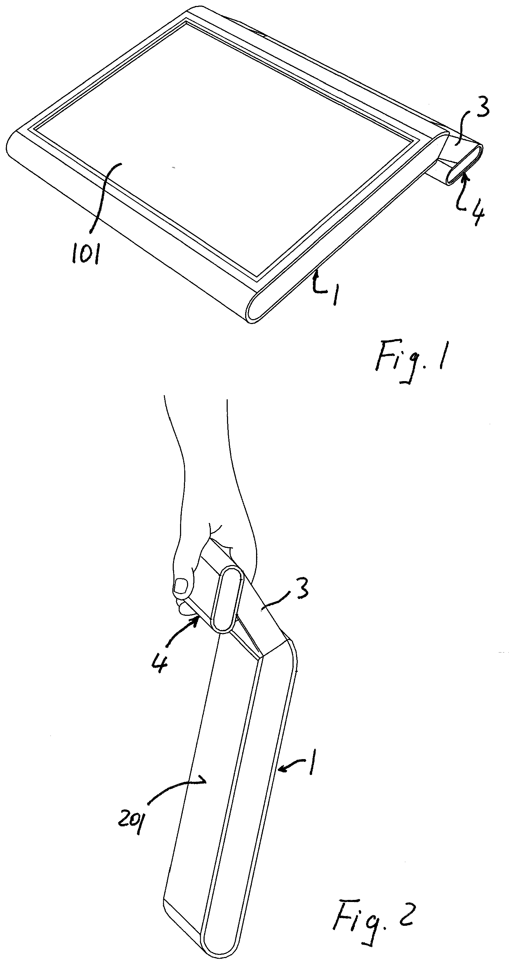

Referring to figures 1 to 4, a portable ultrasound system comprises a main unit and a probe 2 which is connected to the main unit by means of a cable 5.
The probe 2 and the cable 5 can be connected to the main unit by means of usual connectors, which are not illustrated in detail since these connectors are known and widely used for connecting ultrasound probes.
The main unit comprises the hardware and software and the peripherals which are necessary for driving the probe 2 for transmitting ultrasound beams in a body under examination and for receiving ultrasound beams from the said body and which are necessary for processing the received signals in order to generate diagnostic images of the inner parts of the body under examination.

The hardware of the main unit is housed in a case 1 which has the form of a so called portable tablet PC. In particular the case is formed by a thin parallelepiped case having a front and a back side which are the largest sides. The front side is formed approximately entirely by a flat screen, particulalrly a LCD touch screen, 101, such as a LCD screen.

In the example illustrated the front and the back sides are rectangular. Although this is the preferred embodiment, the invention is not intended to be limited to the present rectangular form.
The lateral sides connecting the front and the back sides are the smaller sides of the case.

Two arms 3 protruding from two corners at the ends of one lateral side carry at their ends an elongated member 4. These two arms departs from the opposite ends of one lateral side or from an edge of the back side or from two opposite lateral sides and are oriented in the direction of the back-side of the case 1 and protrude backwards of the said back side, terminating at a certain distance from the back side. Thus the elongated member lies at a certain distance backwards relatively to the back side of the case 1 of the main unit.

Preferably the two arms 3 have identical lengths, so that the elongated member is positioned parallel to the back side of the case and also parallel to one edge of the said back side.

In the present example, the elongated member is parallel to the longer side of the rectangular back side of the case.

Furthermore, the two arms are inclined in such a way that the elongated member is carried also in a position in which the said elongated member is laterally spaced from the lateral edge of the back side of the cage, so that the said elongated member does fall outside the boundaries of the back side.

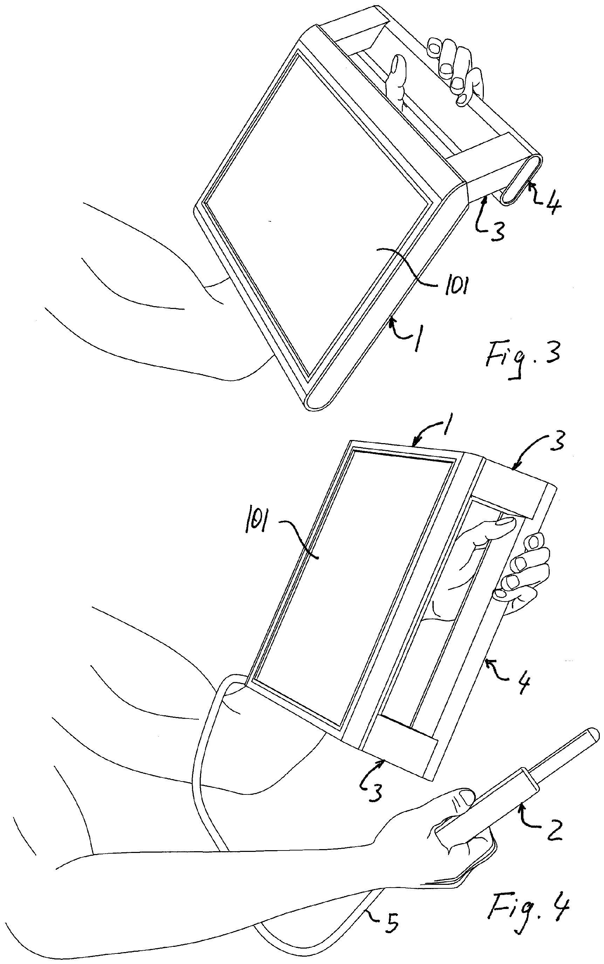

The elongated member has a cross section which is such that it can grasped and held by one hand as illustrated in figures 3 and 4.

As it appears from the figures 1 to 4, the particular configuration of the elongated member and its position relatively to the case 1 of the main unit allow that the said handle has several functions. As illustrated in figure 1, the elongated member 4 has the function of holding the case inclined when it is placed on a table, so that the screen at the front side 101 of the case is inclined in a way ameliorating the sight of the screen. As illustrated in figure 2, the elongated member serves as a handle for carrying the ultrasound system in a way similar to a briefcase.

As illustrated in claims 3 and 4, the elongated member 4 serves as a handle for blocking the case of the main unit in place and supporting the weight of the said case by means of the same arm the hand of which is grasping the elongated member 4.

According to a preferred embodiment the length and the width of the case 1, i.e. the lengths of the edges of the front and back side of the case 1 are such that they are not longer as the mean length of the fore arm approximately from the wrist to the hollow of the elbow.

This allows when the front and back sides are rectangular that the case 1 can be held either in the portrait or in the landscape orientation of the rectangular monitor. When the configuration of the main unit is such that the elongated member is positioned parallel to the longer side of the front and back side of the case, than when the hand holds the elongated member horizontally or approximately horizontally, the monitor has a landscape orientation and the longest side of the case opposite to the one at the elongated member rests on the forearm.

In figure 4 the hand holds the elongated member 4 in a vertical plane and the monitor has a portrait orientation, which means with the longest side in a vertical direction or in a vertical plane, while the lower shorter side of the case which is parallel to the lower shorter edge of the monitor rests on the forearm. In the configuration of figure 3 the arm is oriented along a direction which is approximately parallel to an axis which is perpendicular to the elongated member and parallel to the back side. Preferably the fore arm is approximately coincident with the central axis of the back side which axis is perpendicular to the elongated member 4.

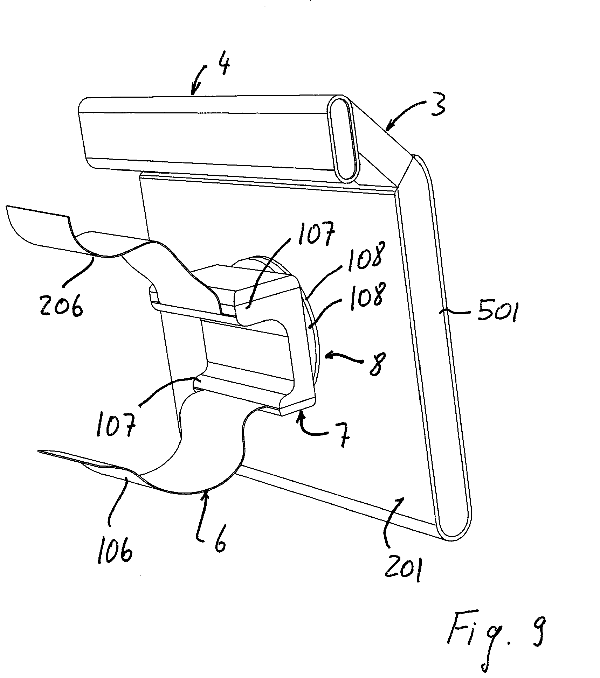

In figure 4, the arm is oriented approximately along a diagonal of the back side of the case 1.
As it appears from fig 9, according to a variant, the case 1, may be provided on its back side 201 with means for securing the case to the fore arm.

The simples means that can be provided are one or more parallel straps that can be tightened around the forearm. For example a strap 6 having a certain width has two ends 106, 206 which carry means for connecting together the said two ends and for tightening the strap. This means can be for example of the kind of the so called velcro tape, one part of which is provided on one end 106 and the other part of the velcro strap is provided on the other end 206 of the strap 6.

Other kinds of connecting and tightening device can be provided as for example the means used for the straps of the diving masks or for the belts of the backpacks.

Because the back side 201 of the case 1 is flat, according to a further feature, the strap or the straps 6 are provided in combination with an anatomical contact part 7. This part is illustrated in figure 9 and is of the saddle like kind, having an U shaped cross section and a form and dimensions allowing the forearm to fit therein at least for a part of its cross section. The ends of the strap 6 departs each one from one of the arms 107 of the U cross section of the saddle like contact part.

According to a further feature, the said saddle like contact part 7 is secured to the back side 201 of the case 1 by means of a device allowing its rotation along an axis perpendicular to the said back side 201. In particular the rotation axis of the said saddle like contact part coincides with the central axis perpendicular to the said back side 201 of the case 1. Many ways can be provided for allowing the saddle like contact part 7 to rotate. One example of device 8 for allowing the rotation of the saddle like contact part 7 is illustrated in figure 9 and has two circular plates 108 secured together at their centre in such a way that the two plates can rotate one relatively to the other. One plate being secured to the back side 201 of the case while the other plate is secured to the saddle like contact part 7. Further more between the two plated there can be provided means for provisionally stopping the two plates in certain angular positions one relatively to the other. This means are not illustrates since many solutions are possible which lie within the opportunity choice of the skilled person. As an example, one plate could be provided on a circular path coaxial with the axis of rotation with one or more notches or cuts which are distributes along the said circular paths, while the other plate is provided on the same circular path with one or more balls, which are housed in a hole and which are pushed permanently with a certain force against the other plate by mean of an elastic element such as a helical spring. When during relative rotation one with respect to the other one of the balls is coincident with one of the said notches the rotation is stopped and can be prosecuted only by applying a certain release force, which is needed in order to push back the ball in the hole and free again the movement of the plates.

Thanks to the fact that the case can be secured tightly to the fore arm, the hand must not anymore grasp tightly the elongated member and is merely for further ensuring that the device will not slip away and fall down and also for balancing the device on the forearm. Thus the hand can be used to activate one or more keys or buttons or the like which may be present on the elongated member and which can be serve for general purposes or for activating special functions during the execution of a scanning so that the hand holding the probe 2 is not necessary and the examination must not be stopped for entering the command or control needed.

The elongated member is advantageously empty inside at least partially and made in such a way that it forma the housing of one or more battery packs or other hardware.

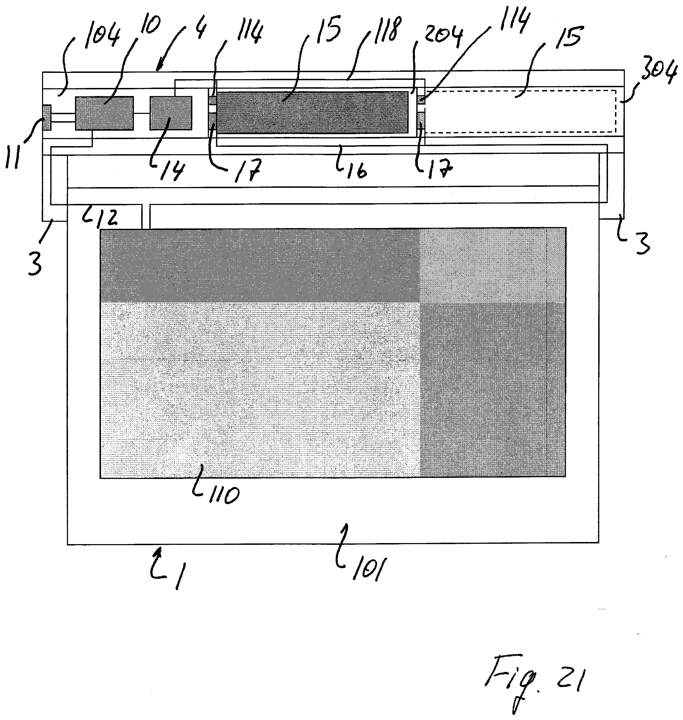

An example of this construction is illustrated in figure 21. The figure is very schematic since the constructive arrangements are matter falling within the technical general knowledge of the skilled person.

Figure 21 represents only one example of several different configurations which are obvious once one configuration is disclosed.

The illustrated elongated member has three separate chambers. Chamber 104 is destined to housing a power feeding circuit indicated with 10 and which is connecter with its input to a connector 11 at one external side, particularly one head side of the elongated member 4. The output of the power feeding unit 10 is connected to the hardware indicated by 110 in the case 1 by means of a power feeding line 12 which passes from the elongated member 4 inside the case 1, by passing through one arm 3 connecting the elongated member 4 to the case 1. In the same chamber 104 or in another separated chamber (non illustrated) a further circuit can be provided which can be for example a battery charger 14. The battery charger 14 can be fed with power by the power feeding unit 10, while its output is connected to a charging connector 114 provided in each of two battery packs housing chambers 204, 304. In this case it is assumed that the battery packs 15 has charging inputs and power outputs which are separated and for which in the corresponding housing 204, 304, there are provided connectors 114 and 17 for automatically connecting a charging line 18 and a power feeding line 16 to the charging input and to the power feeding output of the battery pack 15 when it is inserted in the corresponding chamber 204, 304.

In the present example the two chambers 204, 304 can house each one a battery pack independently one from the other. So the ultrasound system has two so called battery bays in which a battery pack can be inserted independently if a battery pack is present or not in the other bay.

An alternative configuration may consist in the fact that the power feeding unit and the charger are an external device as in the presently known notebook computers, while the chamber 104 is a further battery housing.

The chamber 204, 304 are constructed in such a way to house more than one battery pack 15.

The chambers 104, 204, 304 have an open side through which they can be accessed, A cover can be provided for each of the said chambers. There might be provided that the cover is for closing the open side of all or of part of the chambers.

A further alternative which can be provided consist in the fact that the battery packs 15 are stably housed inside the elongated member 4 and that their cannot be separated therefrom. When the battery packs have to be substituted the entire elongated member has to be substituted. In this case, the elongated member can be secured to the case 1 or to the two arms 3 in releasable manner.

Also the other variant in which the elongated member 4 forms chambers for housing battery packs or hardware and which housings can be accessed for substituting the battery packs or the hardware may be provided with an elongated member which is releasable from the case or from the two arms 3. In this case this may be important for changing the elongated member in order to mount one elongated member which ca house more battery packs or which has different configuration of keys on it or which has different hardware housed in it.

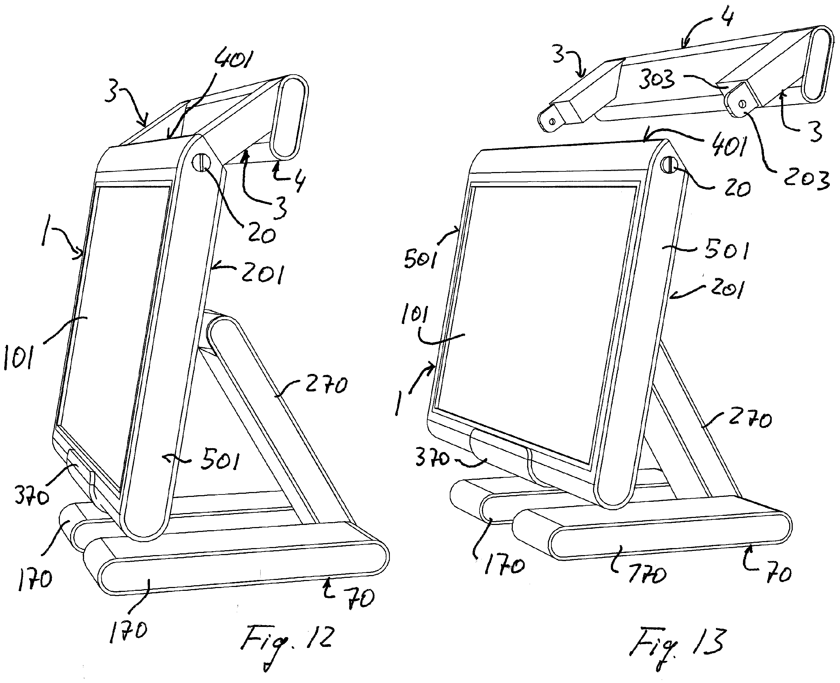

Several different examples are illustrated in figures 12 to 15 and 19 and 10.

In figure 12 to 15, the two arms 3 which support the elongated member 4 in a cantilevered way from the case 1 of the main unit has two terminal protrusions 103 at the end opposite to the one connected to the elongated member. Each protrusion is provided with a passing hole 203. The case 1 of the main unit has at each end of its upper lateral side 401 parallel to which the elongated member 4 has to be held an opening for accessing a pocket which extends parallel to the lateral side 501 which is perpendicular to the one 401 parallel to which the elongated member 4 has to be held and which pocket is dimensioned in such a way that the corresponding protrusion 103 can be inserted therein. The walls forming the lateral sides 501 has a passing hole coinciding with the hole 203 of the protrusion 103. The said hole can pass the pocket from side to side in such a way as to be provided into both walls delimiting the said pocket and one of which walls is formed by the wall of the lateral side 501 of the case 1. The said hole can be threatened along its entire length or only for the part provided in the lateral wall of the pocket which is internal to the case 1. Alternatively the hole 203 in the protrusion 103 is threatened. A screw 20 is provided for being screwed into the hole in the lateral walls of the pocket or in the hole 203 of the protrusion 103 of the arm carrying the elongated member 4.

Relatively to the electric lines for feeding the power signal from the power feeding unit and/or from the battery packs in the elongated member and/or for feeding other signals from other hardware provided in or on the elongated member 4 to the hardware in the case of the main unit, as already illustrated diagrammatically in figure 21, these lines may pass from the elongated member 4 into the case 1 through the arms 3. In the present example of figures 12 to 15, the arms has a bigger cross section than the one of the protrusions 103 and are made tubular. The ends 303 may carry or be conformed as an electric multipolar connector part cooperating with a complementary electric, multipolar connector part which is carried at the lateral side 401 parallel to the elongated member beside the corresponding pocket for the protrusion 103 of each one of the arm 3.

The connector parts will engage each other automatically when the protrusions 103 are inserted in the pockets and are disconnected automatically when the elongated member 4 with the arms 3 is separated from the case 1 of the main unit.

The particular construction of the connector is not illustrated since there are currently available a very large number of electric connectors which can serve to achieve the above function and the skilled person only has to make a choice between the available connectors.

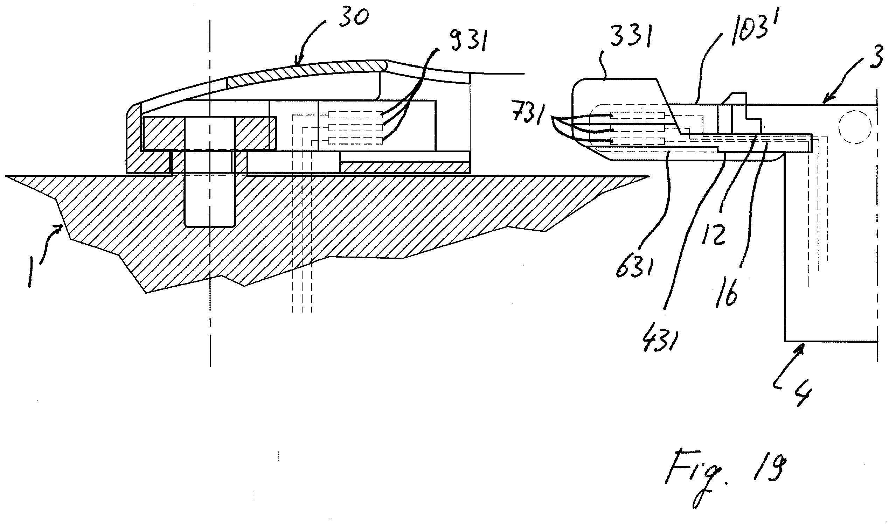

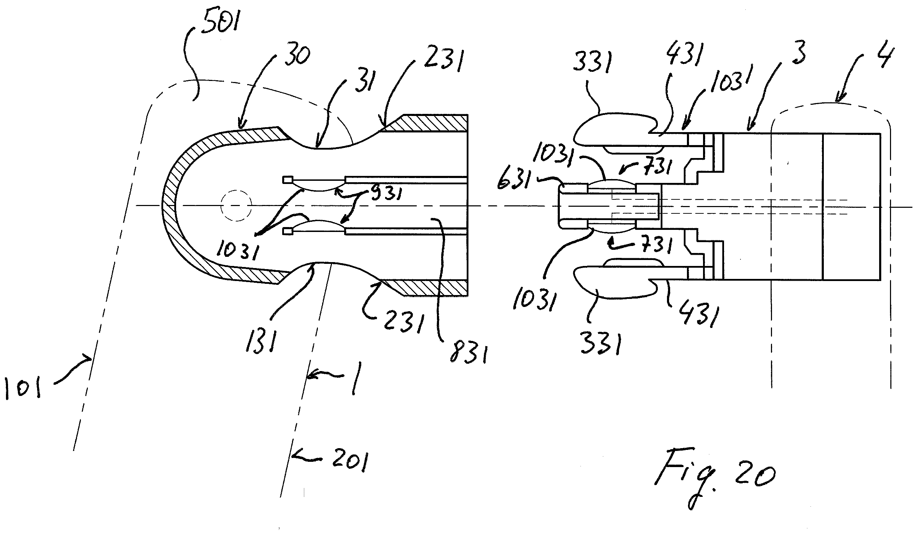

Figure 19 and 20 show a particular which is an alternative to the above embodiment. Here the connection between the arms 3 carrying the elongated member 4 and the case 1 are of the snap in type.

Also in this case there could be plenty of constructions and the one of figures 19 and 20 represents only one example. In this example the connection is of the kind known for example for the belts or for the shoulder straps of the back packs or for the securing straps of the professional fins having an open shoe part.

A pocket like part 30 is secured to each wall of the opposed lateral sides 501 oriented perpendicular to the elongated member 4 and parallel to the arms 3. The pockets has an opening which is oriented towards the arms 3. Each arm 3 has a protrusion 103' which is destined to be inserted in the pocket-like part 30.

The pocket like part 30 has two openings 131 in each of its lateral sides which are perpendicular to the side 501 to which they are secured. The said openings forms an engaging edge 231 for a corresponding engaging tooth 331 which is carried at the end of an elastic tongue 431 of the protrusion 103'. The said protrusion has two elastic tongues 431 which are oriented parallel to the direction of insertion in the pocket 30 and which are eccentric and symmetrically positioned relatively to a central longitudinal axis of the protrusion which is a central axis parallel to the direction of insertion in the protrusion 103' in the pocket part 30. Each elastic tongue 431 is flexible in a direction perpendicular to the said longitudinal central axis and against the said longitudinal central axis, and carry the tooth 331 on its lateral side facing the lateral side of the pocket part 31. In their normal rest position the tongues are oriented in such a way that the rear fronts of the teeth are aligned with the engaging edge 231 of the openings 131 in the lateral sides of the pocket part 30. The end of each tooth is bevelled in such a way as to form a sliding surface of the lateral side of the opening of the pocket part 30 along the said tooth, so that automatically the tongues 431 are bent one against the other allowing the protrusion 103' to enter the pocket like part 30 and slide therein till each one of the two tooth 331 are coincident with the corresponding opening 131 in the lateral wall of the pocket like part and the tongues are free to spring up laterally outwards. Thereby each tooth is automatically engaged in the opening 131 and its rear side is aligned with the retaining edge 231 of the corresponding opening 131.

For freeing the protrusion 103' it is sufficient to press with the hands the teeth one against the other and displace them below the retaining edge so that the protrusion 103' can slip out form the pocket.

A particular multipolar electric connector can be provided in combination of this kind of mechanical connector. The protrusion 103' has a central longitudinal element 631 one of the lateral outer walls or both the lateral outer walls of the said longitudinal element 631 carry several longitudinal electric contacts 731 each one connected to a conductor which passes inside the said central longitudinal element 631 and through the corresponding arm 3 inside the elongated member 4. The pocket like part 30 is provided with a central guide 831 for the central longitudinal element 631, which central guide is a channel like and has lateral walls each of which carry a corresponding number of electric contact 931 which have such a position that each contact will be coincident and will be in contact with a corresponding electric contact 731 on the central longitudinal element 631 of the protrusion 103' when the said protrusion 103' is inserted in the pocket like part 30 in its end position. Each contact 931 is connected to the hardware in the case 1 by means of a conductor passing inside the case 1 through passages in the pocket like part 31 and in the lateral wall 501 to which this part is secured.

Advantageously as indicated in the drawings the electric contacts 731 and 931 are of the kind having an elastic contact terminal 1031 which is urged elastically in a position protruding outwards from the surface of the central longitudinal element 631 and from the lateral walls of the central guiding channel 831 in which the central longitudinal element 631 of the protrusion 103' of the arm 3 slides when the said protrusion is inserted in the pocket like part 30. This protruding elastic contact terminals 1031 can have an arched form so that they are invited to slide one over the other when the electric contacts 731 and 931 comes to slide one over the other.

As it appears from the figures 12 to 15, the present releasable elongated member 4 allows to provide different kinds of elongated elements having the same arms 3 and the same kind of protrusions 103, 103' so that different kinds of elongated elements 4 can be mounted on the case 1.

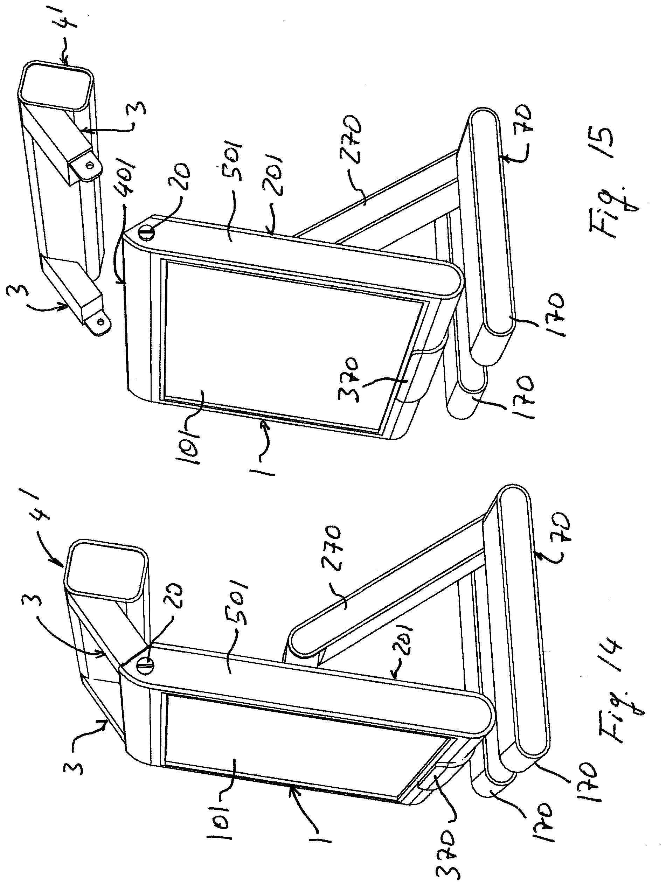

Here for example the two elongated elements 4 of figures 12 and 13 and of figures 14 and 15 differ one from the other in the fact that they have different cross sections. The elongated element of figures 14 and 15 has a cross section which is approximately double in size of the area than the cross section of the elongated member of figures 12 and 13. This allows to have a bigger number of chambers or a bigger chamber for housing more battery packs and/or more hardware in the elongated member 4 and/or also more keys or buttons or commands. When the not a long endurance of the battery packs is needed the thinner elongated element 4 and be mounted on the case. When more endurance or more power is needed the thicker elongated member can be mounted on the case. Also if different additional functions such as for example communication hardware and/or additional driver units for writing and reading memory supports or additional hard disks units or other devices are needed a particularly configured elongated member carrying the said additional devices can be mounted on the case 1.

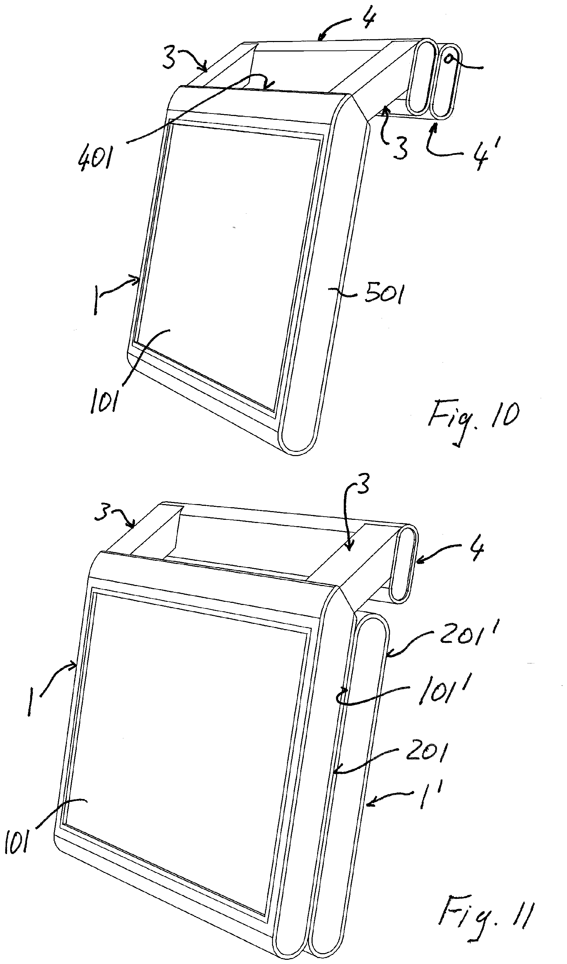

According to a further embodiment of the ultrasound system which is illustrated in fig. 10, a second elongated member 4' can be attached to a first elongated member 4 which is attached to the case 1 by means of the arms 3 either in a stable or in a detachable or releasable manner as described above. The second elongated member 4' serves as a case for further battery packs or further hardware according to the different constructions disclosed above for the first elongated member 4. In this case the power feeding lines and/or other communication lines of the hardware housed in the second elongated member 4' for connecting the battery packs and/or the hardware housed in the said second elongated member to the hardware in the case of the main unit passes through the first elongated member 4. This is done by means of an electric connector of the kind used for connecting a docking station with its notebook computer. In particular a two part connector is provided, a first of the said connector parts being provided on the side of the first elongated member 4 against which a facing side of the second elongate member 4' come into contact when the said second elongated member is attached to the said first one. A second connector part is provided on the said side of the second elongated member facing the side of the first elongated member 4 against which the said second elongated member 4' will come into contact with the said first elongated member 4.

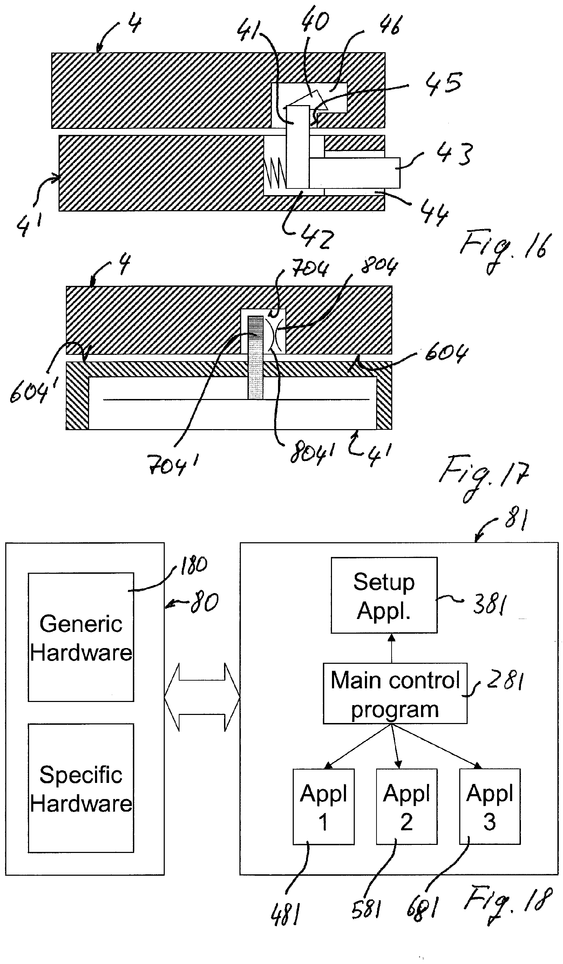

Particularly advantageous is a connector as the one illustrated in figure 17. here the side 604 of the first elongated member 4 has a slot 704 in which a plurality of contact terminals 804 are aligned forming a row of contacts which are oriented with their surfaces of contact to the contact terminals 804' of the second elongated member in a plane perpendicular to the side 604 of the first elongated member 4, i.e. perpendicular to the side of the said first elongated member against which a facing side 604' of the second elongated member 4' is brought in contact when the two elongated member are attached one to the other.

The second elongated member 4' is provided in a position coincident with the said slot with a connector plate 704' carrying a row of contact terminals 804'. The contact terminals 804' are oriented parallel with the contact terminals 804 of the connector part provided on the first elongated member 4 and laterally shifted in such a way that each one of the contact terminals in the slot 704 of the first elongated member 4 will slide against a corresponding one of the contact terminals 804' on the connector plate protruding out of the side 604' of the second elongated member 4'. The contact plate 704' being parallel to the slot and to the surface in the slot 704 carrying the row of contact terminals 804. Each contact terminal 804, 804' can be further formed by a flexible tongue which is arched along an axis perpendicular to the direction of engagement of the contact plate 704' in the slot 704. The arched tongues of the contact terminals 807 in the slot protrudes in the direction of the arched contact terminals 804' on the connector plate 704' of the second elongated member 4' such that the arched tongues of the contact terminals 804 and the one of the contact terminals 804' interfere one against the other, so that each contact terminal 804 and the corresponding contact terminal 804' are pressed elastically one against the other in the engaged condition of the two parts of connector.

When bringing the second elongated member against the first elongated member 4 by aligning the two connector parts the said ones will be automatically engaged one in the other.

The first elongated member 4 can be provided with a further power line for the additional battery packs of the second elongated member 4' or the output of the power lines in the second elongated member are connected to the power lines of the first elongated member 4 so that an extension of the already present power line network is generated.

Similarly the communication lines of the additional hardware housed in the second elongated member can be connected to separate communication lines already provided in the first elongated member 4 and which are dedicated to the said additional hardware or the communication lines provided in the second elongated member 4' can be connected with the communication lines of the first elongated member 4 forming an extension of a communication network which is common to every additional hardware in both the two elongated members 4, 4'.

It is to be noted that not only two elongated members 4, 4' can be attached one to the other mechanically and electrically, but also three or more additional elongated members can be attached in a cascade way one to the other and to the first elongated member.

The mechanical attachment can be made by means of releasable means such as the ones illustrated as an example in figure 16. One of the two elongated members, and in particular the second or further one 4' carries on the side to be brought into contact with a side of the first elongated member 4 a hook 40 which is provided on a pin 41 protruding perpendicularly out of a housing 42 through an opening of the said housing on the side of the second elongated member 4' destined to come into contact with a side of the first elongated member 4. the pin 41 is carried by a cursor 43 which is mounted slidably in a guide 44. The guide 44 being oriented with its longitudinal axis perpendicular to one of the head sides of the elongated member where it is open allowing to the end of the cursor 43 to protrude out of the said opening and being operable by mean of the fingers. An elastic element maintains the cursor 43 in a position in which it protrudes out of the opening of the guide 44 and the hook 40 at the end of the pin 41 is in a position of engagement of a retaining edge 45 which is formed by a undercut in a lateral wall of a notch 46. the notch 46 is provided in the side wall of the first elongated member 4 destined to be brought into contact with a wall of the second elongated member 4' when the two elongated members are attached one to the other. The notch 46 and the hook 40 being provided in coincident positions when the two elongated members are attached one to the other. The hook has preferably a rounded head which helps to be automatically driven for engaging the retaining edge 45 when the two elongated member 4, 4' are brought with their facing sides into contact one with respect to the other.

In order to release the two elongated members one from the other the hook 40 can be disengaged from the retaining edge 45 of the slot 46 by pushing on the head of the cursor 43 protruding out of the opening of the sliding guide 44.

The above is merely an example for demonstrating that mechanical releasable attachment means cen be provided for connecting mechanically the two elongated member one to the other.

The second elongated member 4' can have the same form as the first elongated member 4.

A further variant which can be provided separately or in combination with anyone of the previously disclosed combination of features consist in the provision of a second case 1' having the form and the dimensions of the case 1 of the main unit and which has the function of providing further housings for further battery packs and or further housing for further hardware.

Despite the shape and the dimensions which are such that the second case 1' can be releasable attached with its front side 101' to backside 201 of the case 1 of the main unit, similar features can be provided for this second case 1' as the one disclosed for the second elongated member 4'. Indeed the way and the construction which apply for connecting mechanically one case to the other and for generating electric connections of the battery packs of the additional case 1' and or of the additional hardware to the hardware of the main unit housed in the case 1, can be the same as the one disclosed by means of figures 16 and 17 for the second elongated member. Relatively to the construction of the additional case 1' with reference to the housings for the battery packs and for additional hardware similar features can be provided as the ones illustrated and described for the elongated members in figure 21.

The present embodiment further enhances the possibility and flexibility of the configuration of the device relatively to the endurance of the power supply and to the weight.

Similarly top the construction of the elongated member 4, more than only one additional case 1 can be provided each one of the said additional cases carry each the hook means on one side and the notches on the other side so that a second additional case can be attached to the back side 201' of a first additional case which in turn is attached to the case 1 of the main unit.

According to still another feature, the saddle like contact part 7 can be secured to the back side of the case in a releasable way thanks to releasable attachment means which can be of any kind, being many different attachment means known to the skilled person.

This allows to connect the case 1 to several other supporting devices which enables a correct or optimised positioning of the device.

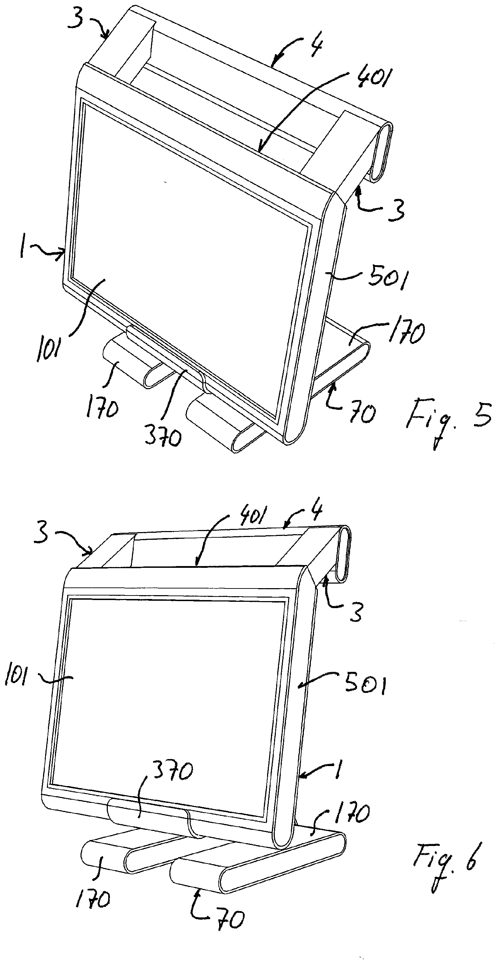

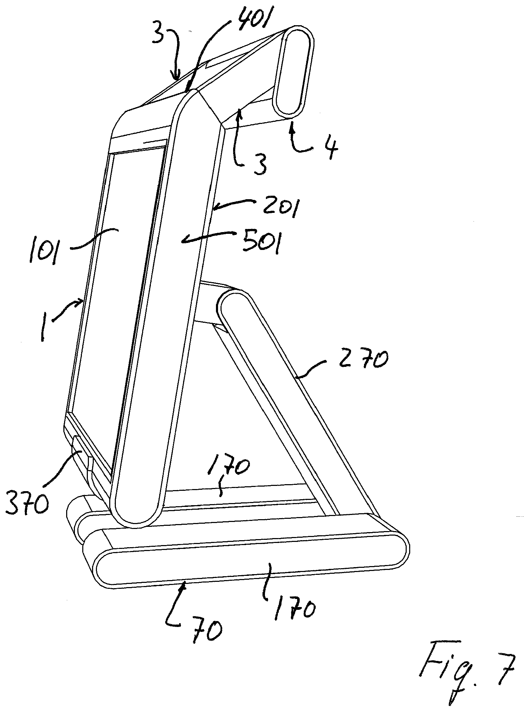

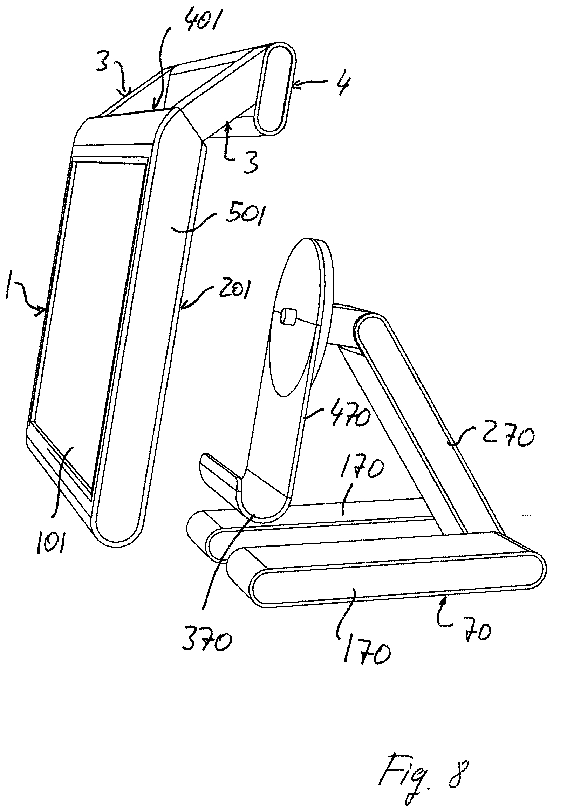

According to figures 5 to 8 and 12 to 15, a table base 70 is provided in combination with the case 1 of the main unit which allows to position the case 1 with the front side formed by the monitor screen substantially in a vertical position. The table base has two horizontal feet 170 which are linked with one of their ends to the end of an intermediate supporting lever 270. at its end the supporting lever 270 carries a back plate 470 to which a lower supporting saddle 370 is secured in a rotatable manner around an axis which is perpendicular to the back plate 470. The lower supporting saddle 370 is provided at such radial distance from the axis of rotation that when the case 1 of the main unit is positioned with its lower horizontal side in the said saddle 370, the axis of rotation is approximately coincident with the central axis perpendicular to the back side of the case 1. the saddle 370 extends till to the said central area of the back side of the case 1 at which it is linked in the rotatable way to the back plate 470 and at which it has releasable connecting means to the back side of the case 1.

Thanks to this construction while lying on the table base, the case 1 can be freely rotated and the user is able to orient the monitor screen in a landscape or in a portrait position.

The hardware driving the monitor of the main unit can advantageously have automatic means for detecting the orientation of the screen of the monitor relatively to the said landscape or portrait orientation and which means automatically adapts the image visualized on the screen to the orientation of the said screen. This means are known and are for example gravity sensors.

Both in the case of the table base 70 and of the saddle like contact part 7 further box like cases may be provided which can be mounted releasably in an intermedite position between the back side of the case 1 and the side of the table base or of the saddle like connection part 7 facing the said back side 201 of the case 1. Each box like case can carry different hardware or further battery packs. One or more of the said box like cases can be provided in such a way that a row of the said one or more boxlike cases secure one to the back of the other are mounted to the back side of the case 1 and to the said row of box like cases the table base or the saddle like connection part are attached.

Here the releasable way of fixing the intermediate boxlike cases can be the same as the one described above for the two or more elongated members 4, 4' or the two or more cases 1, 1' according to the configuration of figures 10 and 11 and 16 and 17.

According to a further feature of the present invention, the portable ultrasound system of the above kind has a completely graphical user interface. In this case the screen being of the touch screen kind and being able to receive commands by touching the screen at different control keys, buttons, switches and cursors being in the form of graphic symbols representing the said keys, buttons, switches and cursors. Selecting means are provided for selecting at least one specific application within a list of application which are available on the portable echographic system, means being provided for loading and executing a specific graphic user interface program which generates on the screen the images of only the keys, buttons, switches and cursors that are needed for carrying out the tasks related to the selected specific application.

In the portable echographic system different electronic units for carrying out specific tasks are substituted by a central processing unit able of executing different programs and of carrying out different tasks, at least a memory being provided in which there are saved different programs for controlling the central processing unit in order to carry out different specific tasks related to the functions of an echographic system, one or more of the said different programs for carrying out specific tasks being loaded by the central processing unit and executed by it when a certain specific application has been selected by the user.

Figure 18 is a simplified block diagram illustrating this structure. The structure is a typical hardware/software architecture. The hardware 80 comprises generic hardware 180 in the form of a processing section able to memorize and execute control programs. The control programs 281, 381, 481, 581 and 681 are of two kinds. Control programs 281 and 381 are general setup programs such as management programs or hardware configuration or setup programs. Control programs 481 to 681 are virtual ultrasound machines, in the sense that routines are provided which when executed by the generic hardware and if necessary by a specific hardware drive the said generic hardware to carry out specific imaging application oriented functions and activate the specific hardware if it is necessary for some very particular application specific functions. Furthermore each control program drives the touch screen in such a way as to generate on it a graphic user interface which comprises icons representing specific keys, buttons, cursors and other controls that are needed for the specific imaging application to which the virtual machine represented by the said control program is directed. The said keys, buttons, cursor icons and the eventually further icons of other kind of manual controls are activable manually by means of the touch screen in such a way as to operate like the corresponding physical hardware devices.

So for example if only bi-dimensional b-mode imaging is needed the main control program will be provided with a selection/activation function of a corresponding control program which is loaded and executed by the hardware 80. According to the said control program a specific graphic user interface is generated on the screen having active icons of control keys, buttons, cursors and/or other manual control devices for controlling an imaging apparatus capable of only acquiring, processing, visualizing and saving B-mode images.

If for example the application needed relates to the determination of vascular flux, if this specific application is provided in the system, then a particular control program selection and activation button will be available which will charge in the hardware 80 a corresponding control program which relates of a corresponding virtual ultrasound apparatus having the functions of operating in the so called Doppler or Power Doppler-mode, other functions which are not needed will be not present in the virtual ultrasound apparatus specialized for determining blood flux.

This are only two examples that can serve to the skilled person to understand the basic idea of the hardware/software structure of the present ultrasound system. Starting from this the skilled person is able to construct any kind of specific application oriented machine and then the control program for generating the corresponding virtual machine.

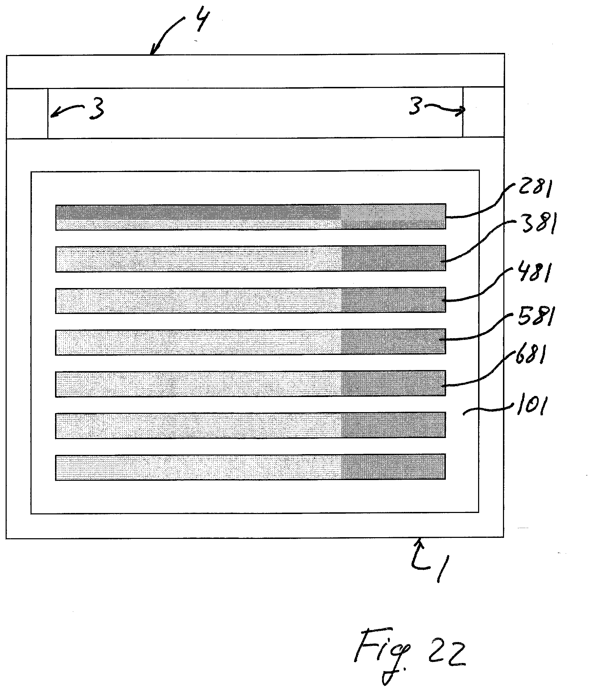

Figure 22 illustrates diagramatically the appearance of an example of the graphic user interface of a main control program.

Here on the touch screen 82 a list of options appears each one of the options being associated to a selection and activation representation of a virtual key or button and each one of the said virtual key or button correspond to an active area of the touch screen which when touched will cause the main control program to address the specific application control program corresponding to the option selected and to load this control program in the operative memory of the hardware which will execute this application specific control program.

In figure 22 seven selection and activation keys are shown as the graphic user interface of the main control program. Two of this corresponds to the setup control programs which are indicated with 281 and 381 in fig. 21 such as the power management setup and the hardware configuration setup. The other five keys are related to five different application specific configurations of an ultrasound system.

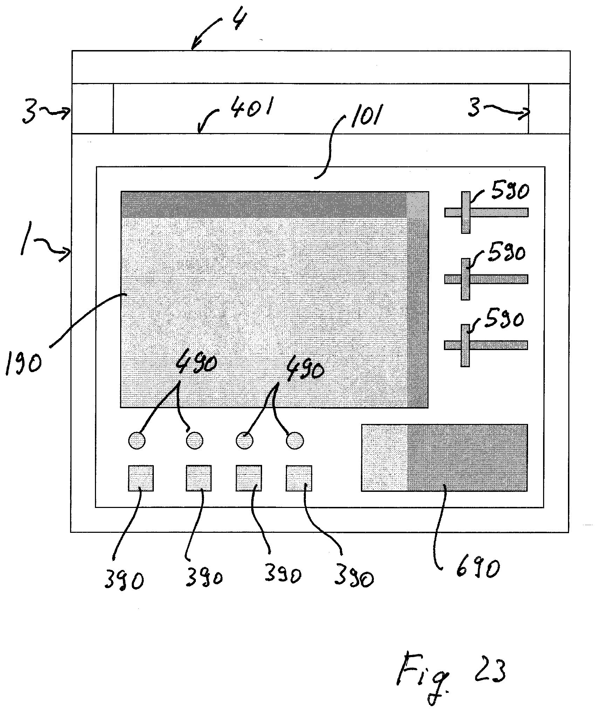

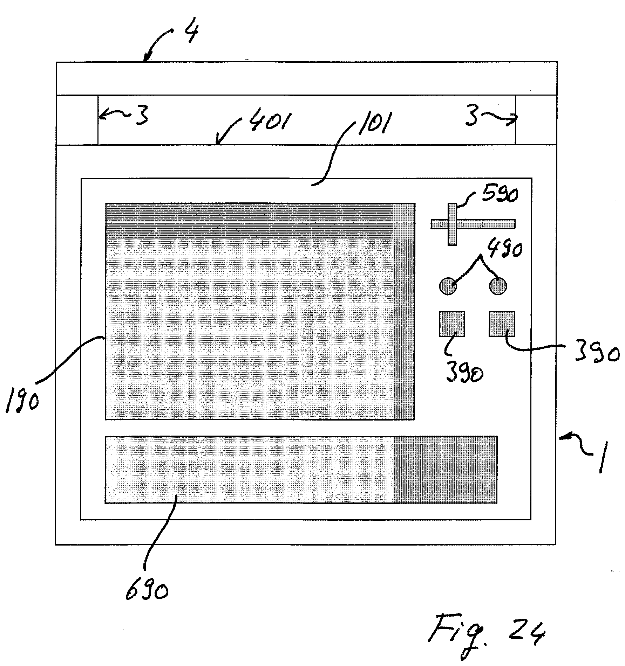

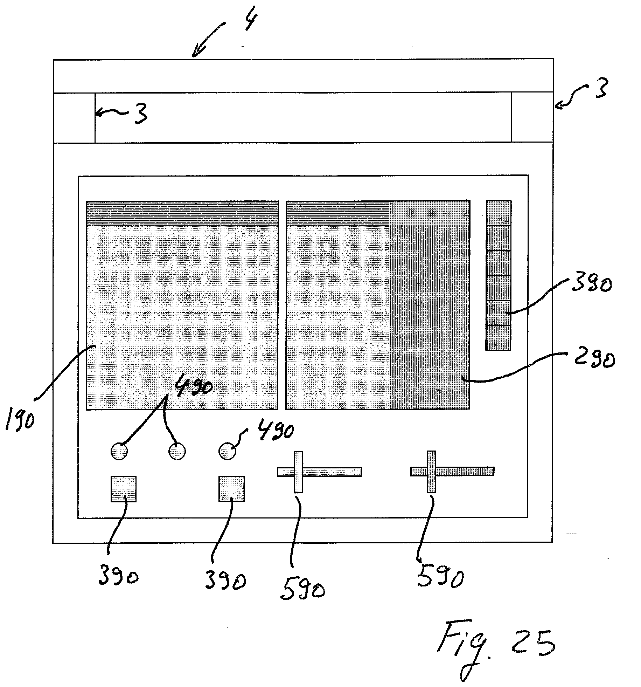

Figures 23 to 25 illustrate three different graphic user interfaces corresponding to three different virtual machines generated by three different application specific control programs such as for example the ones indicated with the numeral 481, 581 and 681 in figure 21.

As it appears clearly the graphic user interface printed on the touch screen comprises different visualisation areas of one or two images indicated by 190, 290; different number of keys indicated with the numerals 390 and different number of signal lights indicated by the numeral 490; different number of cursors indicated by the numeral 590. Further more the keys 390, the lights 490 and the cursor 590 has different positions on the area of the touch screen as also the image visualisation areas 190, 290. Further areas 690 of the screen 90 can be provided for visualizing further informations in the form of an alphanumeric text.

The advantage of the above described architecture are first of all that there is no mechanical control to be activated or driven by the user, so that there are no problem of long term damages of mechanical devices. Furthermore, a hardware configuration would necessitate that a certain number of keys buttons, levers, or other manually activable control means have to be present which for a specific application would be superfluous. Using a graphic user interface the configuration of the manually driven control means can be changed every time in a way which is specific to the imaging application to be carried out so that the configuration of the machine representing the ultrasound system can be limited every time to the provision of the very essential manual controls and thus to the very essential functions.

In addition to the fact that it is possible to spare in the dimensions of the touch screen, in the computational power and in the memory space and thus in the electric power consumption, every graphic user interface corresponding to a certain application specific configuration of the ultrasound system, is very simple to be used and it is also very simple to learn to use the application specific ultrasound system, since the keys, buttons, cursors and the other commands are limited to the ones needed for controlling the application specific machine selected.

In loading the different control programs corresponding each one to an application specific configured ultrasound apparatus, the generic hardware is controlled to carry out functions which in the currently known devices is carried out by dedicated hardware. Only very specific functions will need specific hardware which will have its corresponding software to be correctly driven in combination with the functions carried out by the generic hardware controlled by the control program.

Relating to the above description it has to be stressed out that only some examples are considered among a great number of possible choices.

A possible list of applications for each one of which a specific virtual machine is provided in the form of a specific control program which generates also a dedicated layout of controls which is optimized relatively to the specific application comprises the following applications:
Regional Anaesthesia including Nerve Blocking and Vascular Access;
Veterinary including large and small animals;
Advanced Vascular including Blood Vessel Thickness and Stiffness;
Musculoskeletal including wound View, Sports Medicine and Phlebology;
Esthetical surgery including Fat Thickness and Botox treatment.

The provision of application specific layout of the control interfaces with the apparatus which limits the controls to the ones which are really necessary for controlling the application specific tasks and functions, allows also to generate very simple tutorials which are in the form of executable tutorial programs and which are dedicated each one to a specific application. Particularly each tutorial is a complete demos of the way of using the ultrasound system in the application specific configuration. Furthermore because of the fact that the application specific control programs does not need a huge amount of memory and computational power, each one of the said programs can also be provided with an online held which can print on the screen help messages either automatically or by a request command.

Coming to the detail of the hardware, the generic hardware can be of the kind of a processing unit such as a computer motherboard with integrated video and audio chip, a communication bus with hard drives. A PCI bus for connecting several peripherals, USB and or Parallel and/or serial and/or network communication hardware and ports a solid state work memory for loading the control programs to be executed and a processor. The touch screen is connected to the video chip and has an own processor a so called GPU (graphic processing unit). According to a further feature of the present invention, in order to limit the size and expenses of the hardware and also the weight of the device without loosing computational power, some routines of the control programs representing the virtual apparatuses and which are functional units consisting in a software which drives the generic hardware to carry out the functions of the corresponding unit, are generated so to be carried out by the GPU instead of the CPU (central processing Unit). A particular example consists in the fact that a software scan-converter is provided which is the software version of the currently used hardware scan converter. This software scan converter is executed by the GPU using zero CPU resources. Since the GPU has a considerable computational power which is normally not completely exploited in this case some computational work is passed from the CPU to the GPU so that the CPU is free to carry out other tasks.

## Claims

1. Portable ultrasound system, having only a completely graphical user interface for inputting manual commands,
the said system comprising:
a probe (2) for transmitting and receiving an ultrasonic signal to and from a region of interest;
a main unit comprising circuitry in communication with said probe (2) for forming an image of the region of interest based on the ultrasonic signal; and a display (101) for displaying the formed image; the said main unit having a weight which can be supported by one hand or arm; the said probe (2) being connected to the said circuitry in the main unit,
**characterized in that**
the main unit consists in one single case (1) showing the form of a flat parallelepiped in which the front side is formed substantially entirely by said display (101) which is a so called touch screen and the circuitry for forming an image of the region of interest based on the ultrasonic signal being contained in the said case (1); and the said case (1) of the main unit being provided in combination alternatively with
a releasable attachment member (6, 7, 8) for said main unit case, said attachment member being mountable around a forearm of the system user such that said display (101) is visible to the system user during use;
a releasable handle (3, 4);
a releasable table base (70).

2. Portable ultrasound system according to one or more of the preceding claims, **characterised in that** a monitor screen is provided which is of the touch screen kind and of such kind being able to receive commands by touching the screen;
the different control keys, buttons, switches and cursors or other kind of hand operable control means (390, 490, 590) are in the form of graphic symbols representing the said keys, buttons, switches and cursors and other hand operable control means;
selecting means being also provided for selecting at least one specific application within a list of application (281, 381, 481, 581, 681) which are available on the portable echographic system;
and means being provided for loading and executing a specific graphic user interface program which generates on the screen the images of only the keys, buttons, switches and cursors and of other hand operable control means (190, 290, 390, 490, 590, 690) that are needed for carrying out the tasks related to the selected specific application.

3. Portable ultrasound system according to claim 2, **characterized in that** different electronic units for carrying out specific tasks are substituted by a central processing unit able of executing different control programs and of carrying out different tasks, at least a memory being provided in which there are saved different programs for controlling the central processing unit in order to carry out different specific tasks related to the functions of an echographic system, one or more of the said different programs for carrying out specific tasks being loaded by the central processing unit and executed by it when a certain specific application has been selected by the user.

4. Portable ultrasound system according to claim 3, **characterized in that** a main control program is provided which is loaded and executed by the central processing unit which main control program prints on the display screen the list of applications available (281, 381, 481, 581, 681) with the said portable system and a further graphics user interface comprising the icons of keys, buttons, switches, cursors or similar control devices related to general functions (190, 290, 390, 490, 590, 690), such as a menu for addressing the specific diagnostic or surgical applications and or menus for addressing configuration and adjustments options for the device which are of general purpose, and/or menus for carrying out maintenance or managing tasks of the apparatus and of the devices integrated in it such as file manager routines, memory drives control and maintenance routines, electronic units configuration and diagnostics, driver upgrade, system upgrade and further typical tasks needed for managing and for maintaining the efficiency of a computer system;
the said main control program addresses then depending on the choice made with the help of the menus the programs for carrying out the specific tasks related to the applications or configuration and adjustment options chosen by the user.

5. Portable ultrasound system according to claims 3 or 4 **characterized in that** a further control program is saved in the memory and is available for being executed by the central processing unit, which program consist in an application specific tutorial for the user which is already provided in the echographic system in a ready to start condition;
and **in that** the main control program prints on the screen a graphic user interface with a key icon which addresses the said tutorial program and which key when touched by the user determines that the main control program loads the tutorial program and to execute the said program by the central processing unit.

6. Portable ultrasound system according to one or more of the preceding claims in which in order to spare electric power and maintain the weight limited without affecting the computational power of the system needed by the hardware for executing the programs and carrying out the tasks of the system, a part of the tasks to be carried out by the central processing unit (CPU) are carried out by the graphic processing unit (GPU), particularly a software for causing the processing unit to carry out functions of a scan converter is provided which software is executed by the GPU.

## Patentansprüche

1. Tragbares Ultraschallsystem, das nur eine völlig grafische Benutzeroberfläche zur Eingabe manueller Befehle hat,
wobei das System umfasst:
eine Sonde (2) zum Senden und Empfangen eines Ultraschallsignals zu und von einem interessierenden Bereich;
eine Haupteinheit, die eine Schaltungsanordnung in Kommunikation mit der Sonde (2) zum Erzeugen eines Bildes des interessierenden Bereichs basierend auf dem Ultraschallsignal umfasst; und
eine Anzeigevorrichtung (101) zum Darstellen des gebildeten Bildes; wobei die Haupteinheit ein Gewicht hat, das von einer Hand oder einem Arm getragen werden kann;
wobei die Sonde (2) mit der Schaltungsanordnung in der Haupteinheit verbunden ist,
**dadurch gekennzeichnet, dass**
die Haupteinheit aus einem einzigen Gehäuse (1) besteht, das die Form eines flachen Quaders aufweist, in dem die Vorderseite im Wesentlichen vollständig von der Anzeigevorrichtung (101), einem so genannten Touchscreen, gebildet ist und die Schaltungsanordnung zur Erzeugung eines Bildes des interessierenden Bereichs basierend auf dem Ultraschallsignal in dem Gehäuse (1) aufgenommen ist; und
das Gehäuse (1) der Haupteinheit wahlweise in Kombination mit einem lösbaren Befestigungselement (6, 7, 8) für das Haupteinheitsgehäuse vorgesehen ist, wobei das Befestigungselement um einen Unterarm des Systemnutzers derart montierbar ist, dass die Anzeigevorrichtung (101) für den Systemnutzer im Gebrauch sichtbar ist;
ein lösbarer Griff (3, 4);
eine lösbare Tischbasis (70).

2. Tragbares Ultraschallsystem nach einem oder mehreren der vorhergehenden Ansprüche,
**dadurch gekennzeichnet, dass**
ein Monitorbildschirm vorgesehen ist, der vom Touchscreen-Typ ist und in der Lage ist, Befehle durch Berührung des Bildschirms entgegenzunehmen;
die verschiedenen Bedientasten, Buttons, Schalter und Cursors oder eine andere Art von handbetätigbaren Steuermitteln (390, 490, 590) in Form von grafischen Symbolen vorliegen, die Bedientasten, Buttons, Schalter und Cursors und andere handbetätigbare Steuermittel darstellen;
Auswahlmittel vorgesehen sind, zur Auswahl mindestens einer spezifischen Anwendung innerhalb einer Applikationsliste vorgesehen (281, 381, 481, 581, 681) die auf dem tragbaren Echographiesystem zur Verfügung stehen;
und Mittel zum Laden und Ausführen eines bestimmten grafischen Benutzerschnittstellenprogramms vorgesehen sind, welches auf dem Bildschirm nur die Bilder der Tasten, Buttons, Schalter und Cursors und von anderen handbetätigbaren Steuermitteln (190, 290, 390, 490, 590, 690) erzeugt, die zur Durchführung der mit der ausgewählten spezifischen Anwendung zusammenhängenden Aufgaben benötigt werden.

3. Tragbares Ultraschallsystem nach Anspruch 2,
**dadurch gekennzeichnet, dass**
verschiedene elektronische Einheiten zur Ausführung bestimmter Aufgaben sind durch eine zentrale Verarbeitungseinheit ersetzt, die in der Lage ist, unterschiedliche Steuerprogramme und unterschiedliche Aufgaben auszuführen ist, wobei mindestens ein Speicher vorgesehen ist, in dem verschiedene Programme zur Steuerung der zentralen Verarbeitungseinheit gespeichert sind, um verschiedene spezifische Aufgaben in Bezug auf die Funktionen eines Echographiesystems durchzuführen, wobei ein oder mehrere der unterschiedlichen Programme zur Ausführung bestimmter Aufgaben durch die zentrale Verarbeitungseinheit geladen und von ihr ausgeführt werden, wenn eine bestimmte spezifische Anwendung durch den Benutzer ausgewählt wurde.

4. Tragbares Ultraschallsystem nach Anspruch 3,
**dadurch gekennzeichnet, dass**
ein Hauptsteuerprogramm vorgesehen ist, das von der zentralen Verarbeitungseinheit geladen und ausgeführt wird, welches Hauptsteuerprogramm auf dem Anzeigebildschirm die Liste der zur Verfügung stehenden Applikationen (281, 381, 481, 581, 681) mit dem genannten tragbaren System ausgibt sowie eine weitere grafische Benutzeroberfläche mit den Icons von Tasten, Buttons, Schalter, Cursors oder ähnlicher Steuerelementen, die sich auf allgemeine Funktionen (190, 290, 390, 490, 590, 690) beziehen, wie ein Menü zur Adressierung der spezifischen diagnostischen oder chirurgischen Anwendungen und/oder Menüs zur Adressierung von Allzweck-Konfigurationsoptionen und Einstelloptionen für die Vorrichtung, und/oder Menüs zur Durchführung von Wartungs- oder Verwaltungsaufgaben der Vorrichtung und der darin integrierten Einrichtungen, wie z. B. File-Manager-Routinen, Steuer- und Wartungsroutinen für Speichertreiber, elektronische Einheitskonfiguration und Einheitsdiagnose, Treiber-Upgrade, System-Upgrade und weitere typische Aufgaben, die zur Verwaltung und zur Aufrechterhaltung der Effizienz eines Computersystems benötigt werden;
wobei abhängig von der mit Hilfe der Menüs getroffene Auswahl das Hauptsteuerprogramm die Programme zur Ausführung der spezifischen Aufgaben in Bezug auf die vom Benutzer gewählten Applikationen oder Konfigurations- und Einstelloptionen adressiert.

5. Tragbares Ultraschallsystem nach Anspruch 3 oder 4,
**dadurch gekennzeichnet, dass**
in dem Speicher ein weiteres Steuerprogramm gespeichert ist und zur Ausführung durch die zentrale Verarbeitungseinheit zur Verfügung steht, wobei das Programm in einem anwendungsspezifischen Tutorial für den Benutzer besteht, der bereits in dem Echographiesystem in einem startbereiten Zustand vorgesehen ist;
und dass das Hauptsteuerprogramm auf dem Bildschirm eine grafische Benutzeroberfläche mit einem Tastensymbol ausgibt, das das Tutorial-Programm adressiert, so dass bei Berührung dieser Taste durch den Benutzer bewirkt wird, dass das Hauptsteuerprogramm das Tutorial-Programm lädt und dieses Programm von der zentralen Verarbeitungseinheit ausgeführt wird.

6. Tragbares Ultraschallsystem nach einem oder mehreren der vorhergehenden Ansprüche,
in dem, um elektrische Leistung zu sparen und das Gewicht klein zu halten, ohne die von der Hardware zur Ausführung der Programme benötigte Rechenleistung des Systems zu beeinflussen, und um die Aufgaben des Systems auszuführen, einen Teil der von der zentralen Verarbeitungseinheit (CPU) auszuführenden Aufgaben von der graphischen Verarbeitungseinheit (GPU) durchgeführt werden, und insbesondere eine Software zur Veranlassung der Verarbeitungseinheit zur Ausführung von Funktionen eines Scan-Converters vorgesehen ist, wobei die Software von der GPU ausgeführt wird.

## Revendications

1. Système d'échographie portable, n'ayant qu'une interface utilisateur complètement graphique pour la saisie de commandes manuelles, ledit système comprenant :
une sonde (2) pour la transmission et la réception d'un signal ultrasonore vers et à partir d'une région d'intérêt ;
une unité principale comprenant des circuits en communication avec ladite sonde (2) pour la formation d'une image de la région d' intérêt sur la base du signal ultrasonore ; et
un écran (101) pour l'affichage de l'image formée ;
ladite unité principale ayant un poids qui peut être supporté par une main ou un bras ;
ladite sonde (2) étant connectée auxdits circuits dans l'unité principale ;
**caractérisé en ce que** ladite unité principale unique est constituée d'un seul boîtier (1) possédant une forme parallélépipédique plate, dans laquelle le côté avant est occupé presque entièrement par ledit écran (101) qui est un écran tactile, les circuits pour la formation d'une image de la zone d'intérêt sur la base du signal ultrasonore étant contenus dans ledit boîtier (1) ; ledit boîtier (1) de l'unité principale étant prévu alternativement en association avec un élément de fixation détachable (6, 7, 8) pour ledit boîtier d'unité principale, ledit élément de fixation étant montable autour d'un avant-bras de l'utilisateur du système de façon que ledit écran (101) soit visible à l'utilisateur du système pendant l'emploi ;
une poignée détachable (3, 4) ;
une tablette de base détachable (70).

2. Système d'échographie portable selon l'une ou plusieurs des revendications précédentes, **caractérisé en ce qu'**il comporte un moniteur de type écran tactile, et de type capable à recevoir des commandes par une touche sur l'écran ;
les touches, les boutons, les interrupteurs et les curseurs de commande ou d'autres types de moyens de commande (390, 490, 590) pouvant être actionnés à la main sont en forme de symboles graphiques représentant lesdites touches, et lesdits boutons, interrupteurs, et curseurs et les autres moyens de commande pouvant être actionnés à la main ;
des moyens de sélection étant également prévus pour sélectionner au moins une application spécifique à l'intérieur d'une liste d'applications (281, 381, 481, 581, 681) disponibles sur le système d'échographie portable ;
et des moyens étant prévus pour charger et exécuter un programme d'interface utilisateur graphique spécifique, générant sur l'écran les images seulement des touches, des boutons, des interrupteurs et des curseurs de commande et d'autres types de moyens de commande pouvant être actionnés à la main (190, 290, 390, 490, 590, 690) qui sont nécessaires à accomplir les tâches relatives à l'application spécifique sélectionnée.

3. Système d'échographie portable selon la revendication 2, **caractérisé en ce que** les unités électroniques différentes pour accomplir des tâches spécifiques sont remplacées par une unité centrale de traitement capable d'exécuter des programmes de commande différents et d'accomplir des tâches différentes, au moins une mémoire étant prévue, dans laquelle des programmes différents sont stockés pour contrôler l'unité centrale de traitement afin d'accomplir des tâches spécifiques différentes relatives aux fonctions d'un système d'échographie, l'un ou plusieurs desdits programmes différents pour accomplir des tâches spécifiques étant chargés par l'unité centrale de traitement et exécutés par celle-ci lorsque une certaine application spécifique a été sélectionnée par l'utilisateur.

4. Système d'échographie portable selon la revendication 3, **caractérisé en ce qu'**un programme de commande principal est prévu, qui est chargé et exécuté par l'unité centrale de traitement, ledit programme de commande principale imprimant sur l'écran d'affichage la liste des applications disponibles (281, 381, 481, 581, 681) dans ledit système portable et une autre interface utilisateur graphique comprenant les icônes des touches, des boutons, des interrupteurs, des curseurs ou des dispositifs de commande similaires associés à des fonctions générales (190, 290, 390, 490, 590, 690), tel qu'un menu pour invoquer les applications diagnostiques ou chirurgicales spécifiques e/ou des menus pour invoquer des options de configuration et de réglage de type général pour le dispositif, et/ou des menus pour accomplir des tâches d'entretien ou de gestion de l'appareil et des dispositifs intégrés dans celui-ci, tels que des routines du gestionnaire de fichiers, des routines de contrôle et d'entretien de la mémoire, la configuration et la diagnostique des unités électroniques, la mise à nouveau des pilotes, la mise à nouveau du système, et d'autres tâches typiques nécessaires à la gestion et à l'entretien d'un système informatique pour en assurer l'efficacité ;
ledit programme de commande principal invoquant donc, suivant le choix effectué à l'aide des menus, les programmes pour accomplir les tâches spécifiques relatives aux applications ou les options de configuration et de réglage choisies par l'utilisateur.

5. Système d'échographie portable selon la revendication 3 ou 4, **caractérisé en ce qu'**un autre programme de commande est stocké dans la mémoire et disponible pour être exécuté par l'unité centrale de traitement, ledit programme consistant en un didacticiel d'application spécifique pour l'utilisateur, déjà disponible dans le système d'échographie et prêt à démarrer ;
et **en ce que** le programme de commande principal imprime sur l'écran une interface utilisateur graphique avec une icône de touche invoquant ledit didacticiel, et lorsque ladite touche est touché par l'utilisateur elle fait charger le didacticiel par le programme de commande principal, et fait exécuter celui-ci par l'unité centrale de traitement.

6. Système d'échographie portable selon l'une ou plusieurs des revendications précédentes, dans lequel, afin d'épargner de l'énergie électrique et maintenir un poids limité sans affecter la puissance de calcul du système nécessaire pour que le matériel puisse exécuter les programmes et accomplir les tâches du système, une partie des tâches devant être accomplies par l'unité centrale de traitement (CPU) sont accomplies par le processeur graphique (GPU), notamment par la mise en oeuvre d'un logiciel pour faire effectuer à l'unité de traitement les fonctions d'un convertisseur de balayage, ledit logiciel étant exécuté par le GPU.
